(19) Europäisches Patentamt
European Patent Office
Office européen des brevets

(11) Publication number: **0 266 577 B1**

# (12) EUROPEAN PATENT SPECIFICATION

(45) Date of publication of patent specification: **25.09.91** (51) Int. Cl.$^5$: **C07D 295/10**, A61K 31/40

(21) Application number: **87114722.9**

(22) Date of filing: **08.10.87**

The file contains technical information submitted after the application was filed and not included in this specification

## (54) An optically active propanone derivative and process for producing the same and use thereof.

(30) Priority: **09.10.86 JP 239116/86**
**11.08.87 JP 199086/87**

(43) Date of publication of application:
**11.05.88 Bulletin 88/19**

(45) Publication of the grant of the patent:
**25.09.91 Bulletin 91/39**

(84) Designated Contracting States:
**DE ES FR GB IT SE**

(56) References cited:
**EP-A- 0 163 537**
**EP-A- 0 200 942**
**US-A- 3 203 962**

**PATENT ABSTRACTS OF JAPAN, vol. 9, no. 52 (C-269)[1775], 6th March 1985; & JP - A - 59 190 982 (HOKURIKU SEIYAKU K.K.) 29-10-1984**

(73) Proprietor: **NIPPON KAYAKU KABUSHIKI KAISHA**
**11-2, Fujimi 1-chome Chiyoda-ku**
**Tokyo 102(JP)**

(72) Inventor: **Shiozawa, Akira**
**22-7, Horisakicho**
**Omiya-shi(JP)**
Inventor: **Ishikawa, Michio**
**7-10-307, Sakashita-3-chome**
**Itabashi-ku Tokyo(JP)**
Inventor: **Sugimura, Hideo**
**17-1-103, Shimo-3-chome**
**Kita-ku Tokyo(JP)**
Inventor: **Narita, Kazuhisa**
**27-21-303, Hayamiya-4-chome**
**Nerima-ku Tokyo(JP)**
Inventor: **Yamamoto, Hirotaka**
**4-5, Toricho-7-chome**
**Sendai-shi(JP)**
Inventor: **Sakasai, Takeji**
**12-3-2-306, Numakage-2-chome**
**Urawa-shi(JP)**
Inventor: **Ohtsuki, Kazuo**
**17-10, Shimo-3-chome**
**Kita-ku Tokyo(JP)**

**EP 0 266 577 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Kurashige, Shuji**
**26-7, Sakawa-4-chome**
**Urawa-shi(JP)**

(74) Representative: **Türk, Dietmar, Dr. rer. nat. et al**
**Türk, Gille + Hrabal Patentanwälte Brucknerstrasse 20**
**W-4000 Düsseldorf 13(DE)**

## Description

### Field of the invention

The present invention relates to an optically active ℓ-2-methyl-1-(4-trifluoromethylphenyl)-3-pyrrolidino-1-propanone (hereinafter referred to as ℓ-propanone derivative) having in particular a centrally acting muscle relaxing activity, a process for producing the same, and its use in the treatment of spastic paralysis, muscle hypertonia and diseases caused by sickness of brain blood vessel, brain paralysis or spinal sickness.

### Prior art

As β-aminopropiophenone which has a centrally acting muscle relaxing activity, there has been known 2-methyl-1-(4-methyl-phenyl)-3-piperidino-1-propanone (tolperisone, Japanese Patent Publication No. 20,390/65). Tolperisone has widely been used in treatments for spastic paralysis resulting from muscle hypertonia. However, tolperisone, is not always satisfactory in respect to the effect, and to the duration so that improvement thereon is demanded.

From Patent Abstracts of Japan, vol. 9, No. 52(C-269) *[1775]*, 4'-fluoromethyl-3-amino-2-methyl-substituted propiophenone derivatives having a muscle relaxing activity are known which differ structurally from the above ℓ-propanone derivative by containing a $-CH_2F$ group instead of a $-CF_3$ group on the 4'-position of the phenyl ring.

In EP-A-0 200 942 (which belongs to the prior art according to Article 54(3) EPC) the genral use of 2-methyl-1-(4-tri-fluoromethylphenyl)-3-pyrrolidino-1-propanone in the treatment of muscle hypertonia is described. However, this statement refers to the racemate and not to its optically active ℓ-isomer.

### Summary of the invention

Subject-matter of the present invention is an optically active ℓ-2-methyl-1-(4-trifluoromethylphenyl)-3-pyrrolidino-1-propanon and a pharmaceutically acceptable salt thereof.

A further subject-matter of the present invention is a process for producing an optically active ℓ-2-methyl-1-(4-trifluoro-methylphenyl)-3-pyrrolidino-1-propanone, and a pharmaceutically acceptable salt thereof which is characterized by reacting dℓ-2-methyl-1-(4-trifluoromethylphenyl)-3-pyrrolidino-1-propanone with optically active L-acetylphenylglycine or optically active D-malic acid in a solvent at a temperature ranging from room temperature to the boiling point of the solvent to form corresponding two kinds of corresponding diastereomeric salts, subjecting them to optical resolution at a temperature below the boiling point of the solvent and decomposing the deposited crystalline diastereomeric salt in water in the presence of an alkali at 0 to 50°C and isolating an optically active ℓ-2-methyl-1-(4-trifluoromethyl-phenyl)-3-pyrrolidino-1-propanone by extracting it and converting it into a pharmaceutically acceptable salt thereof in a conventional manner, if desired.

According to a preferred embodiment of the present invention the solvent for the optical resolution is at least one solvent selected from the group consisting of a ketone, an alcohol and an aliphatic alkyl carboxylate. The use of ethylacetate as the solvent for the optical resolution is particularly preferred.

The crystal depositing time in optical resolution preferably is 1 to 150 hours and the crystal depositing temperature preferably is 0 to 70°C.

According to a third aspect the present invention relates to a compound as stated above or its pharmaceutically acceptable salt for use in the treatment of (i) spastic paralysis, (ii) muscle hypertonia or (iii) diseases causes by sickness of brain blood vessel, brain paralysis or spinal sickness.

### Detailed description of the invention

The compound of the present invention can be produced, for example, as follows. That is, the racemate of the propanone derivative is reacted with optically active L-acetylphenylglycine or optically active D-malic acid in an appropriate solvent to form corresponding two kinds of diastereomeric salts. The resulting solution is concentrated, cooled, or to the solution is added the solvent which can decrease the solubility of the salt to deposit crystals of a slightly soluble diastereomeric salt. The crystals are separated from the liquid, and the resulting salt from reacting optically active d,ℓ-propanone derivative with the optically active L-acetylphenylglycine or the optically active D-malic acid is decomposed by an aqueous alkali solution, and the optically active ℓ-isomer of the propane derivative can be isolated.

The solvent used in the present invention is not limited, so long as a solvent is used in which both of racemate of the propanone derivative and optically active L-acetylphenyl-glycine or optically active D-malic acid can be dissolved to form diastereomeric salts as well as in which both of the slightly soluble and easily soluble diastereomeric salts can be dissolved at a temperature in the range of from room temperature to the boiling point of the solvent. Then the slightly soluble diasteromeric salt is deposited as crystals by concentration, cooling or addition of an other solvent.

The preferred solvents are ketones such as acetone, methyl ethyl ketone, methyl isobutyl ketone; alcohols such as methanol, ethanol, propanol, isopropanol; and alkyl aliphatic carboxylates, for example $C_1$-$C_4$ alkyl acetate such as methyl acetate, ethyl acetate, butyl acetate. Particularly preferred are ethyl acetate and isopropanol.

The proportion of racemate of the d,ℓ-propanone derivative to optically active L-acetylphenylglycine or optically active D-malic acid to be used is about 0,5 to 1,5 equivalents, preferably about one equivalent, of the latter per equivalent of the former.

The temperature at which the racemate of the d,ℓ-propanone derivative is reacted with optically active L-acetylphenylglycine or optically active D-malic acid is a temperature in the range of from room temperature to the boiling point of the solvent.

The proportion of the amount of the solvent to be used to the amount of the diastereomeric salt may be in a wide range of

from 0,1 time (V/W) to 10 times (V/W), preferred
is 0,1 time (V/W) to 4 times (V/W).

The crystal depositing time is 1-150 hours, preferably 1-60 hours, more preferably 10-50 hours.

The crystal depositing temperature may be below the boiling point of the solvent used, and preferred is a temperature in the range of from 0° to 70° C, particularly 0° to 50° C.

The solvents which decrease the solubility include tetrahydrofuran, ethyl acetate and ethers.

Decomposition of the obtained optically active diasteromeric salt into an optically active ℓ-propanone derivative and an optically active L-acetylphenylglycine or optically active D-malic acid can be carried out in water in the presence of an alkali such as ammonia, sodium bicarbonate, potassium bicarbonate, or diluted aqueous sodium hydroxide solution at 0° to 50° C, preferably at 0° to 30° C. In this case, the amount of alkali may be an equivalent or more per equivalent of the diastereomeric salt.

Isolation of the optically active ℓ-propanone derivative from the decomposition solution can be carried out by extracting it with a nonhydrophylic solvent such as an ether, ethyl acetate, or methylene chloride at 0° C to 40° C, and then distilling off the solvent.

Furthermore, when the decomposition of the salt is carried out in the co-existence of an aqueous alkali solution and the above-mentioned organic solvent, the decomposition and extraction of the optically active ℓ-propanone derivative can be accomplished simultaneously. A free base of the optically active ℓ-propanone derivative thus obtained can be optionally changed into an acid addition salt according to the conventional methods. As exemplary salts can be illustrated salts of inorganic acids such as hydrochloric acid, hydrobromic acid, and sulfuric acid, or salts of organic acids such as acetic acid, citric acid, maleic acid, fumaric acid, and tartaric acid.

When the compound of the present invention is used as a central muscle relaxant, the compound can be administered either orally or parenterally. Although the effective dose varies depending on the condition and age of the patient being treated and the method of administration, it is usually 0.1 - 20 mg/kg/day.

The compound of the present invention is administered in the form of pharmaceutical preparations composed by mixing the compound with an appropriate pharmaceutical carrier. Such pharmaceutical preparations includs tablets, granules, fine grains, powder, capsule, injection, and suppository.

The pharmaceutical activity of the compound of the present invention is described below.

1. Action to ischemic decerebrate rigidity in rats

The effect on the ischemic decerebrate rigidity was investigated by using Fukuda et al method (H. Fukuda, T. Ito, S. Hashimoto and Y. Kudo; Japan. J. Pharmacol. 24, 810 (1974)). Ishemic rigidity is characterized by the rigidity in four limbs, especially in the fore limbs, which is considered resulting from the hyperactivity of α-motoneuro n. This preparation is regarded as an excellent experimental model for spastic paralysis. Medicines having an relaxing effect on this rigidity model are thought as those having a centrally acting muscle relaxing effect.

Rigidity was induced in male Wistar rats (200 -400 g) according to Fukuda et al method, a rat was fixed on its back on a fixing stand after tracheal cannula was inserted under either anesthesia, both of common carotid arteries were ligated and the basilar artery was cauterized with a bipolar coagulator (MICRO-IC,

manufactured by Mizuho Medical Industry Co., Ltd.) to block the blood circulation. The rigidity was recorded as described below.

After rigidity was induced, its forepaws were made to grisp an end of a celluloid plate provided with strain gauges on both sides thereof. A change of the resistance observed when the celluloid plate was forced up by the rigidity of the forepaws was recorded as a tension through a bridge circuit on an ink writing recorder.

A rigidity inhibition rate was calculated according to the following equation:
Ridigity inhibition rate =

$$100 - \frac{\text{Tension after 30 min. from administration}}{\text{Tension before administration}} \times 100\ (\%)$$

The results are shown in the following Table 1.

The compound to be tested was dissolved in distilled water and administered in a dosage of 100 mg/kg through a catheter which had been previously insertred in the stomach.

2. Acute toxicity

By using mice, a 50% fatal dose ($LD_{50}$, mg/kg) of the compound to be tested on intraveous and intragastric administration was determined.

3. Results of the test

The results are shown in Table 1.

Table 1

| Compound ~~No.~~ to be tested | Inhibition rate | $LD_{50}$ Intraveous ad. | $LD_{50}$ Intragastric ad. |
|---|---|---|---|
| ~~1~~ l-isomer of the invention | 51.3% | 107 mg/kg | 620 mg/kg |

The compound of the present invention has a rigidity relaxing effect on the decerebrate rigidity model which is called as a model for some type of spastic paralysis, and is expected to have an excellent treatment effect on the recovery of hyperactivity of muscle tension on spastic paralysis, muscle hypertonia, and various diseases caused by sickness of brain blood vessel, brain paralysis, spinal sickness.

The compounds of the present invention are described below more specifically referring to Referential Example and Examples.

Example 1

l-2-Methyl-1-(4-trifluoromethylphenyl)-3-pyrrolidino-1-propanone hydrochloride

(i) In 150 ml of isopropanol were dissolved 24.7 g of dl-2-methyl-1-(4-trifluoromethylphenyl)-3-pyrrolidino-1-propanone and L-acetylphenylglycine ($[\alpha]_D^{20}$ = 215,9°, c=1.0, ethanol). After the salt was formed, the solution was concentrated under reduced pressure to 60 ml and then allowed to stand

overnight in a refrigerator. The deposited crystalline material was isolated by filtration, and the crude crystalline material was recrystallized from 30 ml of isopropanol to give 13.4 g of L-acetylphenylglycine salt of ℓ-2-methyl-1-(4-trifluoromethylphenyl)-3-pyrrolidino-1-propanone having $[\alpha]_D^{20}$ of 39,3° (c=1.0, methanol).

(ii) In 150 ml of water was dissolved 10.3 g of L-acetylphenylglycine salt of ℓ-2-methyl-1-(4-trifluoromethylphenyl)-3-pyrrolidino-1-propanone obtained in (i). To the resulting solution 150 ml of ethyl ether was added. The solution was neutralized with a 4% aqueous sodium bicabonate solution and extracted with ether. The ether layer was extracted with 1N HCl. The aqueous layer was extracted several times with dichloromethane, and then the dichloromethane layer was dried over anhydrous magnesium sulfate. Subsequently, the solvent was distilled off under reduced pressure, and the residue was recrystallized from dichloromethane-ethyl acetate to give 5.1 g of ℓ-2-methyl-1-(4-trifluoromethylphenyl)-3-pyrrolidino-1-propanone hydrochloride having $[\alpha]_D^{20}$ of -43,3° (c=1.0, methanol) and a melting point of 155° of 157° C.

Example 2

ℓ-2-Methyl-1-(4-trifluoromethylphenyl)-3-pyrrolidino-1-propanone hydrochloride

(i) In 1400 ml of ethyl acetate were dissolved 306 g of dℓ-2-methyl-1-(4-trifluoromethylphenyl)-3-pyrrolidino-1-propanone and 210 g of L-acetylphenylglycine ($[\alpha]_D^{20}$ = +210.0°, c=1.0, methanol). After the salt was formed, the solution was allowed to stand overnight at room temperature and then was stirred at about 5° C for 3 hours. The deposited crystals were filtrated and dried under reduced pressure to give 219 g of first crystals of crude L-acetylphenylglycine salt of ℓ-2-methyl-1-(4-trifluoromethylphenyl)-3-pyrrolidino-1-propanone (yield: 43%, content: about 93%, optical purity: about 98%).

The filtrate was concentrated to 574 g under reduced pressure, allowed to stand at room temperature for 2 days and then stirred at about 5° C for 3 hours. The deposited crystals were filtrated and then dried under reduced pressure to obtain 175 g of second crystals of crude L-acetylphenylglycine salt of ℓ-2-methyl-1-(4-trifluoromethylphenyl)-3-pyrolidino-1-propanone (content: about 85%, optical purity: about 92%).

(ii) To the first and second crystals (total amount: 394 g) prepared in above (i) was added 630 ml of a 10% aqueous sodium chloride solution, 950 ml of ethyl acetate, and 60 ml of aqueous ammonia. The ethyl acetate layer was extracted, washed with 315 ml of a 10% aqueous sodium chloride solution and then dried over anhydrous magnesium sulfate. Subsequently, 29 g of gaseous hydrogen chloride was gradually introduced into the ethyl acetate solution under cooling (25° C or below). The resulting solution was stirred at an inner temperature of about 5° C. The crystals were filtrated, washed with 370 ml of ethyl acetate, and then dried under reduced pressure to give 217 g of ℓ-2-methyl-1-(4-trifluoromethylphenyl)-3-pyrrolidino-1-propanone hydrochloride (content: 99%, optical purity: 97%).

(iii) The ℓ-2-methyl-1-(4-trifluoromethylphenyl)-3-pyrrolidino-1-propanone hydrochloride prepared in above (ii) was treated in the same manner as in (ii) to give highly purified ℓ-2-methyl-1-(4-trifluoromethylphenyl)-3-pyrrolidino-1-propanone hydrochloride with a content of 99.5% or more, an optical purity of 99.5% or more and $[\alpha]_D^{20}$ of -45.0° (c=1.0, methanol) and a melting point of 156° to 159° C.

Example 3

ℓ-2-Methyl-1-(4-trifluoromethylphenyl)-3-pyrrolidino-1-propanone hydrochloride

(i) In 65 ml of ethyl acetate were dissolved 14.3 g (50.0 mmol) of dℓ-2-methyl-1-(4-trifluoromethylphenyl)-3-pyrrolidino-1-propanone and 9.66 g (50.0 mmol) of L-acetylphenylglycine ($[\alpha]_D^{20}$ = +212.8°, c=1.0, methanol). After the salt was formed, the solution was concentrated under reduced pressure to a residual amount of 27 g, and then was allowed to stand at room temperature for 7 days. To the solution was added with 45 ml of a 10% aqueous sodium chloride solution, 87 ml of ethyl acetate and 4.2 ml of aqueous ammonia and the ethyl acetate layer was extracted. The ethyl acetate layer was washed with 35 ml of a 10% aqueous sodium chloride solution and then dried over anhydrous magnesium sulfate. Subsequently, 12.5 ml of a 4N hydrochloric acid/dioxane solution was added to the ethyl acetate solution under cooling (25° C or below). The resulting solution was concentrated under reduced pressure. To the residue was added 85 ml of ethyl acetate. The resulting solution was stirred at room temperature for 1 to 2 hours, and then the crystals were filtrated, and washed with ethyl acetate, and then dried under reduced pressure to give 14.60 g of ℓ-2-methyl-1-(4-trifluoromethylphenyl)-3-pyrrolidino-1-propanone hydrochloride (content: 99%, optical purity: 92%).

(ii) The crude ℓ-2-methyl-1-(4-trifluoromethylphenyl)-3-pyrrolidino-1-propanone hydrochloride prepared in above (i) was treated in the same manner as in Example 2 (ii) and (iii) to give highly purified ℓ-2-methyl-1-(4-trifluoromethylphenyl)-3-pyrrolidino-1-propanone hydrochloride with a content of 99.5% or more, an optical purity of 99.5% or more, $[\alpha]_D^{20}$ of -44.8° (c = 1.0, methanol).

Example 4

ℓ-2-Methyl-1-(4-trifluoromethylphenyl)-3-pyrrolidino-1-propanone hydrochlcride

(i) In 46 ml of hot acetone were dissolved 1.42 g (5.0 mmol) of dℓ-2-methyl-1-(4-trifluoromethylphenyl)-3-pyrrolidino-1-propanone and 0.67 g (5.0 mml) of D-malic acid ($[\alpha]_D^{20}$ = +6.98°, c = 1.0, methanol). After the salt was formed, the mixture was allowed to stand at room temperature. The crude crystals were filtered off and then recrystallized from acetone to give 0.263 g D-malic acid salt of ℓ-2-methyl-1-(4-trifluoromethylphenyl)-3-pyrrolidino-1-propanone having an optical purity of about 96.6% and $[\alpha]_D^{20}$ of -27° to -28° (c = 1.0, methanol).

(ii) The D-malic acid salt of ℓ-2-methyl-1-(4-trifluoromethylphenyl)-3-pyrrolidino-1-propanone prepared in above (i) was treated in the same manner as in Example 2 (ii) and (iii) to give highly purified ℓ-2-methyl-1-(4-trifluoromethylphenyl)-3-pyrrolidino-1-propanone hydrochloride having a content of 99.5% or more and optical purity of 99.5% or more, $[\alpha]_D^{20}$ of -44.8° (c = 1.0, methanol) and a melting point of 158° to 159°C.

Referential Example

1-(4-Trifluoromethylphenyl)-2-methyl-3-pyrrolidino-1-propanone hydrochloride

To a mixture of 2.50 g of 1-(4-trifluoromethylphenyl)-1-propanone, 1.11 g of paraformaldehyde, 1.60 g of pyrrolidine hydrochloride and 20 ml of isopropanol was added 0.1 ml of concentrated hydrochloride acid, and the resulting solution was heated under reflux for 16 hours. After completion of the reaction, the reaction solution was evaporated under reduced pressure to give the residue. To the resulting residue was added with water and washed with ethyl acetate. The aqueous layer was alkalized with aqeous ammonia, and then was extracted with ethyl acetate. The ethyl acetate layer was dried over anhydrous magnesium salfate, and then was evaporated under reduced pressure to give 1.58 g (44.8%) of 1-(4-trifluoromethylphenyl)-2-methyl-3-pyrrolidino-1-propanone as an oil.

IR $\nu_{max}^{neat}$ $cm^{-1}$: 1690

MMR δ($CDCl_3$, TMS): 1.25 (d, 3H, J=7.0Hz,

-COCH($C\underline{H}_3$)$CH_2$-), 1.4-2.1 (m, 4H, 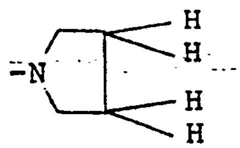 ),

2.3-3.2 (m, 6H, 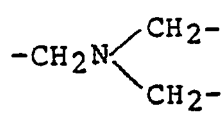 ),

3.65 (1H, m; 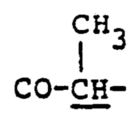 ),

7.70 (2H, d, J = 8.0Hz, aromatic H), 8.05 (2H, d, J = 8.0Hz, aromatic H).

Mass m/z (relative intensity): 285 (2.21, $M^{+}$), 214 (100), 173 (100), 145 (100), 95 (29.7), 84 (100)

This oil was dissolved in ethyl ether and gaseous hydrogen chloride introduced into the solution to give the corresponding hydrochloride salt.

## Claims

### Claims for the following Contracting States : DE, FR, GB, IT, SE

1. An optically active ℓ-2-methyl-1-(4-trifluoromethylphenyl)-3-pyrrolidino-1-propanone and a pharmaceutically acceptable salt thereof.

2. A process for producing an optically active ℓ-2-methyl-1-(4-trifluoromethylphenyl)-3-pyrrolidino-1-propanone, and a pharmaceutically acceptable salt thereof, which is characterized by reacting dℓ-2-methyl-1-(4-trifluoromethylphenyl)-3-pyrrolidino-1-propanone with optically active L-acetylphenylglycine or optically active D-malic acid in a solvent at a temperature ranging from room temperature to the boiling point of the solvent to form corresponding two kinds of diastereomeric salts, subjecting them to optical resolution at a temperature below the boiling point of the solvent and decomposing the deposited crystalline diastereomeric salt in water in the presence of an alkali at 0 to 50° C and isolating the optically active ℓ-2-methyl-1-(4-trifluoromethylphenyl)-3-pyrrolidino-1-propanone by extracting it and converting it into a pharmaceutically acceptable salt thereof in a conventional manner, if desired.

3. The process according to Claim 2, wherein the solvent for the optical resolution is at least one solvent selected from a group consisting of a ketone, an alcohol and an aliphatic alkyl carboxylate.

4. The process according to Claim 3, wherein the solvent for the optical resolution is ethyl acetate.

5. The process according to Claim 2, wherein the crystal depositing time in optical resolution is 1 to 150 hours and the crystal depositing temperature is 0° to 70° C.

6. A compound according to claim 1 or its pharmaceutically acceptable salt for use in the treatment of (i) spastic paralysis, (ii) muscle hypertonia or (iii) diseases caused by sickness of brain blood vessel, brain paralysis or spinal sickness.

### Claims for the following Contracting State : ES

1. A process for producing an optically active ℓ-2-methyl-1-(4-trifluoromethylphenyl)-3-pyrrolidino-1-propanone and a pharmaceutically acceptable salt thereof, which is characterized by reacting dℓ-2-methyl-1-(4-trifluoromethylphenyl)-3-pyrrolidino-1-propanone with optically active L-acetylphenylglycine or optically active D-malic acid in a solvent at a temperature ranging from room temperature to the boiling point of the solvent to form corresponding two kinds of diastereomeric salts, subjecting them to optical resolution at a temperature below the boiling point of the solvent and decomposing the deposited crystalline diastereomeric salt in water in the presence of an alkali at 0 to 50° C and isolating the optically active ℓ-2-methyl-1-(4-trifluoromethylphenyl)-3-pyrrolidino-1-propanone by extracting it and optionally converting it into a pharmaceutically acceptable salt thereof in a conventional manner.

2. The process according to Claim 1, wherein the solvent for the optical resolution is at least one solvent selected from a group consisting of a ketone, an alcohol and an aliphatic alkyl carboxylate.

3. The process according to Claim 2, wherein the solvent for the optical resolution is ethyl acetate.

4. The process according to Claim 1, wherein the crystal depositing time in optical resolution is 1 to 150 hours and the crystal depositing temperature is 0° to 70° C.

5. Use of the compound obtained in any of claims 1 to 4 in the treatment of (i) spastic paralysis, (ii) muscle hypertonia or (iii) diseases caused by sickness of brain blood vessel, brain paralysis or spinal sickness.

## Revendications

### Revendications pour les Etats contractants suivants : DE, FR, GB, IT, SE

1. 1-2-méthyl-1-(4-trifluorométhylphényl)-3-pyrrolidino-1-propanoneoptiquement active et l'un de ses sels acceptable du point de vue pharmaceutique.

2. Procédé de préparation d'une 1-2-méthyl-1-(4-trifluorométhyl-phényl)-3-pyrrolidino-1-propanone optiquement active et l'un de ses sels acceptable du point de vue pharmaceutique, caractérisé par la réaction de la dl-2-méthyl-1-(4-trifluorométhylphényl) -3-pyrrolidino-1-propanone avec la L-acétylphénylglycine optiquement active ou l'acide D-malique optiquement actif dans un solvant, à une température comprise entre la température ambiante et le point d'ébullition du solvant pour former deux formes de sels diastéréoisomériques correspondantes, leur dédoublement optique à une température inférieure au point d'ébullition du solvant et la décomposition du sel diastéréoisomérique cristallin dans l'eau, en présence d'une base, entre 0°C et 50°C, et l'isolement de la 1-2-méthyl-1-(4-trifluorométhylphényl) -3-pyrrolidino-1-propanone optiquement active par son extraction et sa conversion en un sel acceptable du point de vue pharmaceutique selon une méthode classique, si désiré.

3. Procédé selon la Revendication 2, dans lequel le solvant utilisé pour le dédoublement optique est au moins l'un des solvants choisi parmi un groupe comprenant une cétone, un alcool et un carboxylate d'alkyle aliphatique.

4. Procédé selon la Revendication 3, dans lequel le solvant utilisé pour le dédoublement optique est l'acétate d'éthyle.

5. Procédé selon la Revendication 2, dans lequel le temps de dépôt du cristal lors du dédoublement optique est de 1 à 150 heures et la température de dépôt du cristal est comprise entre 0°C et 70°C.

6. Composé selon la Revendication 1 ou son sel acceptable du point de vue pharmaceutique destiné au traitement de (i) la paralysie spasmodique, (ii) l'hypertonie musculaire ou (iii) les maladies causées par accidents vasculaires cérébraux, la paralysie d'origine cérébrale ou une lésion de la moelle épinière.

**Revendications pour l'Etat contractant suivant : ES**

1. Procédé de préparation d'une 1-2-méthyl-1-(4-trifluorométhylphényl) -3-pyrrolidino-1-propanone optiquement active et l'un de ses sels acceptable du point de vue pharmaceutique, caractérisé par la réaction de la dl-2-méthyl-1-(4-trifluorométhylphenyl) -3-pyrrolidino-1-propanone avec la L-acétylphénylglycine optiquement active ou l'acide D-malique optiquement actif dans un solvant, à une température comprise entre la température ambiante et le point d'ébullition du solvant pour former deux formes de sels diastéréoisomériques correspondantes, leur dédoublement optique à une température inférieure au point d'ébullition du solvant et la décomposition du sel diastéréoisomérique cristallin dans l'eau, en présence d'une base, entre 0°C et 50°C, et l'isolement de la 1-2-méthyl1-(4-trifluorométhyl-phényl)-3-pyrrolidino-1-propanone optiquement active par son extraction et sa conversion facultative en un sel acceptable du point de vue pharmaceutique selon une méthode classique.

2. Procédé selon la Revendication 1, dans lequel le solvant utilisé pour le dédoublement optique est au moins l'un des solvants choisi parmi un groupe comprenant une cétone, un alcool et un carboxylate d'alkyle aliphatique.

3. Procédé selon la Revendication 2, dans lequel le solvant utilisé pour le dédoublement optique est l'acétate d'éthyle.

4. Procédé selon la Revendication 1, dans lequel le temps de dépôt du cristal lors du dédoublement optique est de 1 à 150 heures et la température de dépôt du cristal est comprise entre 0°C et 70°C.

5. Utilisation du composé obtenu dans l'une des Revendications 1 à 4 dans le traitement de (i) la paralysie spasmodique, (ii) l'hypertonie musculaire ou (iii) les maladies causées par accidents vasculaires cérébraux, la paralysie d'origine cérébrale ou une lésion de la moelle épinière.

**Patentansprüche**

**Patentansprüche für folgende Vertragsstaaten : DE, FR, GB, IT, SE**

1. Optisch aktives ℓ-2-Methyl-1-(4-trifluoromethylphenyl)-3-pyrrolidino-1-propanon und ein pharmazeutisch verträgliches Salz davon.

2. Verfahren zur Herstellung eines optisch aktiven ℓ-2-Methyl-1-(4-trifluoromethylphenyl)-3-pyrrolidino-1-propanon und eines pharmazeutisch verträglichen Salzes hiervon, welches gekennzeichnet ist durch die Reaktion von dℓ-2-Methyl-1-(4-trifluoromethylphenyl)-3-pyrrolidino-1-propanon mit optisch aktivem L-Acetylphenylglycin oder optisch aktiver D-Apfelsäure in einem Lösungsmittel bei einer Temperatur im Bereich von Raumtemperatur bis zum Siedepunkt des Lösungsmittels um die korrespondierenden beiden Arten von diastereomeren Salzen zu bilden, Unterwerfung dieser der optischen Aufspaltung bei einer Temperatur unterhalb des Siedepunkts des Lösungsmittels und Zersetzung des abgeschiedenen kristallinen diastereomeren Salzes in Wasser in der Gegenwart von Alkali bei 0 bis 50 °C und Isolierung des optisch aktiven ℓ-2-Methyl-1-(4-trifluoromethylphenyl)-3-pyrrolidino-1-propanons durch Extraktion und Umwandlung in ein pharmazeutisch verträgliches Salz davon in üblicher Weise, wenn gewünscht.

3. Verfahren nach Anspruch 2, worin das Lösungsmittel für die optische Aufspaltung wenigstens ein Lösungsmittel ausgewählt aus der Gruppe ist, die aus einem Keton, einem Alkohol und einem aliphatischen Alkylcarboxylat besteht.

4. Verfahren nach Anspruch 3, worin das Lösungsmittel für die optische Aufspaltung Ethylacetat ist.

5. Verfahren nach Anspruch 2, worin die Kristallabscheidungszeitdauer in der optischen Aufspaltung 1 bis 150 Stunden ist und die Kristallabscheidungstemperatur 0 bis 70 °C ist.

6. Verbindung nach Anspruch 1 oder deren pharmazeutisch verträgliches Salz zur Verwendung bei der Behandlung von (i) spastischer Lähmung, (ii) Muskelhypertonus oder (iii) Krankheiten verursacht durch Erkrankung der Gehirnblutgefäße, Gehirnlähmung oder Rückenmarkserkrankung.

**Patentansprüche für folgenden Vertragsstaat : ES**

1. Verfahren zur Herstellung eines optisch aktiven ℓ-2-Methyl-1-(4-trifluoromethylphenyl)-3-pyrrolidino-1-propanon und eines pharmazeutisch verträglichen Salzes hiervon, welches gekennzeichnet ist durch die Reaktion von dℓ-2-Methyl-1-(4-trifluoromethylphenyl)-3-pyrrolidino-1-propanon mit optisch aktivem L-Acetylphenylglycin oder optisch aktiver D-Apfelsäure in einem Lösungsmittel bei einer Temperatur im Bereich von Raumtemperatur bis zum Siedepunkt des Lösungsmittels, um die korrespondierenden beiden Arten von diastereomeren Salzen zu bilden, Unterwerfung dieser der optischen Aufspaltung bei einer Temperatur unterhalb des Siedepunkts des Lösungsmittels und Zersetzung des abgeschiedenen kristallinen diastereomeren Salzes in Wasser in der Gegenwart von Alkali bei 0 bis 50 °C und Isolierung des optisch aktiven ℓ-2-Methyl-1-(4-trifluoromethylphenyl)-3-pyrrolidino-1-propanons durch Extraktion und Umwandlung in ein pharmazeutisch verträgliches Salz davon in üblicher Weise, wenn gewünscht.

2. Verfahren nach Anspruch 1, worin das Lösungsmittel für die optische Aufspaltung wenigstens ein Lösungsmittel ausgewählt aus der Gruppe ist, die aus einem Keton, einem Alkohol und einem aliphatischen Alkylcarboxylat besteht.

3. Verfahren nach Anspruch 1, worin das Lösungsmittel für die optische Aufspaltung Ethylacetat ist.

4. Verfahren nach Anspruch 3, worin die Kristallabscheidungszeitdauer in der optischen Aufspaltung 1 bis 150 Stunden ist und die Kristallabscheidungstemperatur 0 bis 70 °C ist.

5. Verwendung der Verbindung erhalten nach einem der Ansprüche 1 bis 4 zur Behandlung von (i) spastischer Lähmung, (ii) Muskelhypertonus oder (iii) Krankheiten verursacht durch Erkrankungen der Gehirnblutgefäße, Gehirnlähmung oder Rückenmarkserkrankungen.